# EUROPEAN PATENT APPLICATION

(11) **EP 4 641 478 A1**
(43) Date of publication of application: **29.10.2025**
(21) Application number: 23906598.0
(22) Date of filing: 24.11.2023
(51) Int. Cl.: G06Q 50/12, G06Q 50/04, G06Q 50/26

(54) **INFORMATION PROCESSING DEVICE, INFORMATION PROCESSING METHOD, AND INFORMATION PROCESSING PROGRAM**

(30) Priority: 19.12.2022 JP 2022202675
(71) Applicant: Zensho Holdings Co., Ltd., Minato-ku, Tokyo 108-0075 (JP)
(72) Inventor: TAKAHASHI Masaaki, Tokyo 108-0075 (JP); OTSUKA Eiji, Tokyo 108-0075 (JP); SHIRAISHI Keisuke, Tokyo 108-0075 (JP); ABE Etsuko, Tokyo 108-0075 (JP)
(74) Representative: AWA Sweden AB
(86) International application number: PCT/JP2023/042252
(87) International publication number: WO 2024/135229

(57) **Abstract**

Provide an information processing device capable of outputting inspection items of the food and their reference values. The information processing device includes: a storage configured to store inspection information in which a food is associated with inspection items to be inspected for components configuring the food; and a plurality of pieces of reference value information, each of which defines a reference value for each inspection item; a first receiver configured to receive an input of information indicating a food; a second receiver configured to receive an input of information indicating a criterion for performing an inspection; a first extractor configured to extract, on the basis of the food received by the first receiver and the inspection information, the inspection items to be inspected for the food; a second extractor configured to extract, on the basis of the criterion received by the second receiver and the reference value information corresponding to the criterion, a respective reference value for each of the inspection items extracted by the first extractor; and an output unit configured to output inspection target information in which the inspection items extracted by the first extractor are associated with the reference values of the inspection items extracted by the second extractor.

## Description

### BACKGROUND OF THE INVENTION

### 1. Field of the Invention

The present disclosure relates to an information processing device, information processing method, and information processing program.

### 2. Description of Related Art

In restaurants, inspections are conducted for food hygiene management. In a group company that operates multiple stores in a chain, a health officer that performs sanitary supervision may be hard to be assigned to each restaurant due to considerations such as cost management and labor shortages. Accordingly, inspections for sanitary supervision may be performed by causing each store to inquire with a dedicated health officer, for example, regarding methods for food sanitary supervision. However, this places a heavy load on the health officer. Accordingly, the information processing device described in patent document 1 associates the food ingredients with the inspection items needed for the food ingredients and stores them in its storage. Further, the information processing device identifies the food ingredients contained in the dish, refers to the information stored in the storage, and outputs the inspection items in accordance with the identified food ingredients.

### Prior Art Documents

### Patent Documents

Patent Document 1: WO2022/113188

### SUMMARY OF THE INVENTION

### Problems to be Solved by the Invention

Various factors are involved in food risks (food product risks) such as food poisoning. Examples of such factors may include the storage temperature and storage time of the food. As a specific example here, the inspection content for a food may vary between a case where the food is stored at 7°C for 5 days and a case where the same food is stored at 35°C for 6 hours. In addition, the inspection content for the same ingredient may vary between a case where the food is heated before being served for consumption of the dish and a case where the food is consumed at room temperature without being heated. It is also recognized that the sanitation inspections to be performed may vary depending on the location where the food is served.

Accordingly, a limit of the ability of a single health officer to control and notify the contents of the food inspections, which vary in accordance with various factors such as food storage and cooking conditions, is recognized, as in one example above, and a need for such a device exists. The information processing device described in patent document 1 has the problem in that it is not able to provide inspection contents that can vary depending on the location where the inspection is to be performed as described above.

Therefore, it is an object of the present invention to provide an information processing device, an information processing method, and an information processing program capable of outputting the inspection contents of foods in accordance with various criteria.

### Means to Solve the Problems

In order to solve the above problem, an information processing device according to an embodiment includes: a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria; a first receiver configured to receive an input of information indicating a food; a second receiver configured to receive an input of information indicating a criterion for performing an inspection; a first extractor configured to extract, on the basis of the food received by the first receiver and the inspection information, the inspection items to be inspected for the food; a second extractor configured to extract, on the basis of the criterion received by the second receiver and the reference value information corresponding to the criterion, a respective reference value for each of the inspection items extracted by the first extractor; and an output unit configured to output inspection target information in which the inspection items extracted by the first extractor are associated with the reference values of the inspection items extracted by the second extractor.

In order to solve the above problem, an information processing method according to an embodiment causes a computer having access to a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria to perform: a first reception step for receiving an input of information indicating a food; a second reception step for receiving an input of information indicating a criterion for performing an inspection; a first extraction step for extracting, on the basis of the food received in the first reception step and the inspection information, the inspection items to be inspected for the food; a second extraction step for extracting, on the basis of the criterion received in the second reception step and the reference value information corresponding to the criterion, a respective reference value for each of the inspection items extracted in the first extraction step; and an outputting step for associating and outputting the inspection items extracted in the first extraction step with the reference values of the inspection items extracted in the second extraction step.

In order to solve the above problem, an information processing program according to an embodiment causes a computer having access to a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria to embody: a first reception function of receiving an input of information indicating a food; a second reception function of receiving an input of information indicating a criterion for performing an inspection; a first extraction function of extracting, on the basis of the food received by the first reception function and the inspection information, the inspection items to be inspected for the food; a second extraction function of extracting, on the basis of the criterion received by the second reception function and the reference value information corresponding to the criterion, a respective reference value for each of the inspection items extracted by the first extraction function; and an outputting function of associating and outputting the inspection items extracted by the first extraction function with the reference values of the inspection items extracted by the second extraction function.

In the above information processing device, the pieces of criteria may include a first criterion defined by each country, a second criterion defined as a global standard, and a third criterion defined by a predetermined third party, the pieces of reference value information may include first reference value information on the basis of the first criterion, second reference value information on the basis of the second criterion, and third reference value information on the basis of the third criterion, and the second extractor may extract the reference values corresponding to the inspection items extracted by the first extractor on the basis of a priority order defined in an order of the first reference value information, the second reference value information, and the third reference value information.

In the above information processing device, the second receiver may receive input of information on a location where an inspection is to be performed as the information indicating the criterion, and the second extractor may extract a reference value of the first reference value information if the first reference value information corresponding to the information on the location where the inspection is to be performed is available, and extract a reference value of the second reference value information or third reference value information if the first reference value information is not available.

In addition, the above information processing device may further include a positioning unit configured to acquire a current location. The second receiver may accept information indicating the current location acquired by the positioning unit as information indicating a criterion for performing the inspection.

In the above information processing device, a component of the food may be an ingredient used in the food, the inspection item may be information indicating a type of bacteria to be inspected for each ingredient, and the reference value may be information indicating a number or amount of the bacteria per unit.

### Advantageous Effects of the Invention

The information processing device according in one form is capable of outputting information indicating the inspection contents that should be inspected for foods in accordance with various criteria.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
FIG. 1 is a diagram illustrating an example of a system configuration of an inspection system according to an embodiment,
FIG. 2 shows a block diagram illustrating an example of a configuration of an information processing device according to an embodiment,
FIG. 3 is a conceptual data diagram illustrating an example of a data composition of an inspection table indicating food ingredients according to a food and inspection items for each ingredient,
FIG. 4A is a conceptual data diagram illustrating an example of a data composition of a reference value table for a global standard according to an embodiment,
FIG. 4B is a conceptual data diagram illustrating an example of a data composition of a reference value table for a national standard according to an embodiment,
FIG. 4C is a conceptual data diagram illustrating an example of a data composition of a reference value table for a company standard according to an embodiment,
FIG. 5 is a main flowchart illustrating an example of processing related to food inspection of the information processing device according to an embodiment, and
FIG. 6 is a sub-flowchart illustrating an example of processing related to an extraction of reference values for the information processing device according to an embodiment.

### DETAILED DESCRIPTION OF EMBODIMENTS

One embodiment will be described in detail below with reference to the drawings.

### Overview

First, an outline of an information processing device 100 according to an embodiment will be described.

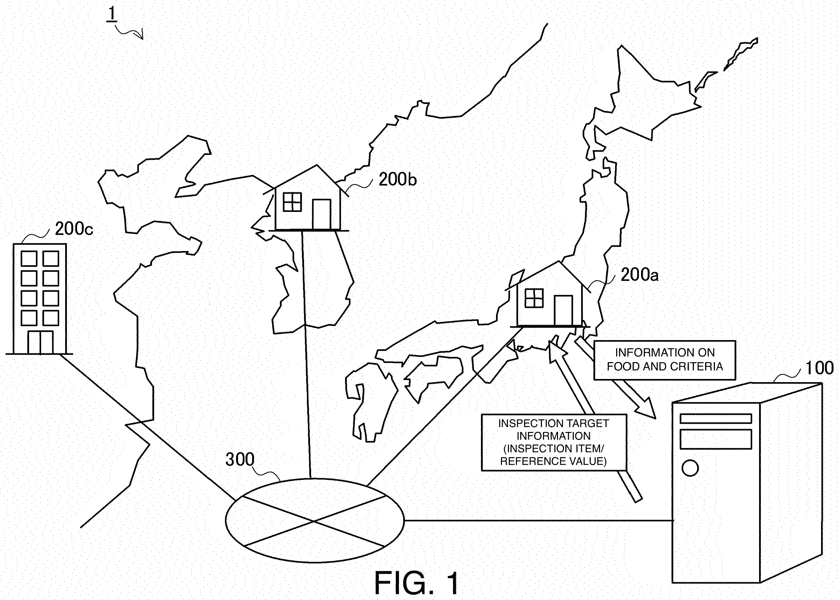

FIG. 1 illustrates an inspection system associated with the information processing device 100 according to the embodiment.

The inspection system 1 includes the information processing device 100 that is connected to a plurality of stores 200a, 200b, 200c via a network 300. Hereinafter, when no special distinction is made with respect to stores, they will be referred to as store 200. Although the term "store" is used here for convenience, to be precise, the information processing device 100 is communicatively connected to the information processing terminals in each of the stores 200 via the network 300.

The information processing device 100 may be directed to a computer such as a server, desktop, laptop, tablet and smartphone, for example. The information processing device 100 may be configured, for example, as an information processing device that supplies inspection details for food hygiene control to each store 200.

As shown in FIG. 1, in the inspection system 1, each store 200 conducts food hygiene inspections for offering foods (products). To this end, information on the food to be inspected and information indicating the criteria for performing the inspection are transmitted from the information processing terminal at each of the stores 200 to the information processing device 100. The information processing device 100 identifies the inspection items to be inspected with respect to the received information on the food, and also identifies the inspection criteria for those inspection items on the basis of the received information indicating the criteria for performing the inspection. The information processing device then associates the inspection items with their reference values and transmits the associated information as specified inspection target information to the information processing terminals of the stores 200 that perform the inspection. With this configuration, the inspection device 1 allows each of the stores 200 to recognize the inspection contents needed at its respective location, thereby enabling the execution of appropriate inspections.

Here, the food can be a dish or an ingredient for a dish. The food may be directed to a product offered to customers at the store 200. The food information may include a dish name indicating the food, or it may be directed to identification information indicating the food on the inspection system 1. The information indicating the criteria for performing the inspection corresponds to information for identifying a reference value for the inspection applicable at the store where the inspection is to be performed, and may be directed to, as an example, information indicating the location of the store or its identification. The inspection is to test whether certain bacteria are adhering to the food outside of the regulations, and whether it is safe for the customers and others of the store 200 to eat.

Although the store 200 is shown here as an example of the location where the inspection is to be performed, this is not limited thereto. The inspection may be performed in locations such as a warehouse in which ingredients used in the store are aggregated, a central kitchen for chain stores in which ingredients are collectively cooked, a processing plant in which the food is processed, or a management facility (or company) in which food storage and management, for example, are performed.

### Configuration of Information Processing Device 100

A detailed description of the information processing device 100 according to an embodiment will be descried in detail.

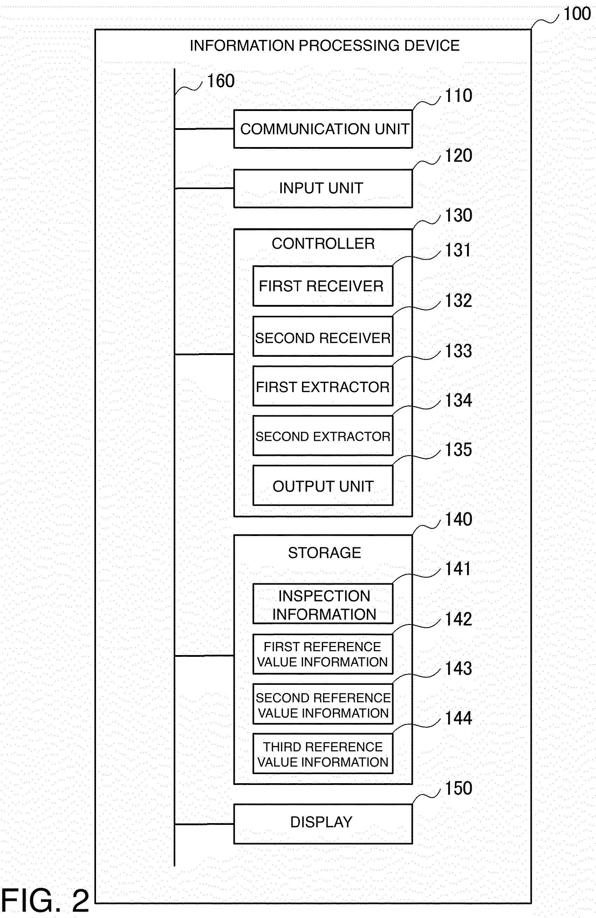

FIG. 2 is a block diagram illustrating the information processing device 100 according to an embodiment.

As shown in FIG. 2, the information processing device 100 includes a communication unit 110, an input unit 120, a controller 130, a storage 140, and a display 150.

The communication unit 110 corresponds to a communication interface that serves to transmit and receive information via the network 300 to and from external devices, for example, information processing terminals provided in the store 200. The communication unit 110 receives information transmitted from the external devices and communicates it to the controller 130. It also transmits the specified information to a specified external device according to instructions from the controller 130. The communication unit 110, for example, receives and communicates to the controller 130 information on the food to be inspected and criteria information indicating the criteria by which the food is to be inspected. In addition, the communication unit 110 transmits, in accordance with the instructions from the controller 130, information indicating the inspection items for the food and their reference values to the information processing terminal provided in the store 200 where the inspection is to be performed.

The input unit 120 corresponds to an interface with the function of accepting input from the user of the information processing device 100 and transmitting the accepted input to the controller 130. The input unit 120 may be embodied by input devices such as a keyboard and mouse, for example, or by a microphone that accepts voice input. The input unit 120 may, for example, accept input of information on the food to be inspected and information indicating inspection criteria, and communicate the information to the controller 130.

The controller 130 is directed to a processor that serves to control the portions of the information processing device 100. The controller 130 can embody various functions to be embodied by the information processing device 100 using various programs and data stored in the storage 140. The controller 130 functions as the information processing device 100 by executing a program for identifying inspection items and reference values stored in the storage 140.

The controller 130 includes a first receiver 131, a second receiver 132, a first extractor 133, a second extractor 134, and an output unit 135 as functions to be performed.

The first receiver 131 accepts input of information indicating food. Here, the food is directed to food for which the inspection is to be performed. The first receiver 131 accepts from the communication unit 110 information on food for which inspection is to be performed at the store 200, for example. The first receiver 131 may also accept information on food from the user of the information processing device 100 through the input unit 120. The first receiver 131 communicates the accepted information indicating the food to the first extractor 133.

The second receiver 132 accepts input of the information indicating the criteria for performing the inspection. The information indicating the criteria for performing the inspection, as described above, corresponds to information that identifies the criteria for inspections suitable for implementation at the store 200, and may primarily correspond to information indicating the location where the inspection is to be performed. If the information processing device 100 is aware of the location of the store 200 in advance, it does not need to accept the information via the communication unit 110 or input unit 120. The second receiver 132 communicates the information indicating the criteria for performing the inspection to the second extractor 134.

The first extractor 133 identifies inspection items to be performed on the communicated food on the basis of the information on the food communicated from the first receiver 131 and the inspection information 141 stored in the storage 140. That is, in the inspection information 141, the inspection item corresponding to the name of the food is identified, and the identified inspection item is communicated to the second extractor 134.

When the second extractor 134 receives the information from the second receiver 132 indicating the criteria for performing the inspection, it identifies the first reference value information 142 that matches the criteria. When the second extractor 134 receives the inspection items from the first extractor 133, it identifies the reference values that the food should satisfy for each inspection item by referring to the identified first reference value information 142, second reference value information 143, and third reference value information 144. Here, the satisfaction of the reference value for the inspection item for the food may basically be directed to a fact that the number of bacteria on the food is below the reference value. The second extractor 134 associates the inspection item with the identified reference value and communicates it to the output unit 135.

The output unit 135 generates inspection target information in which each inspection item communicated from the second extractor 134 is associated with the corresponding reference value, and transmits it via the communication unit 110 to the information processing terminal of the store 200 where the inspection is performed.

The storage 140 serves to store various programs, data, and parameters, for example, needed for the information processing device 100 to operate. The storage 140 may be specifically configured by various recording media such as a main storage configured by a ROM and RAM, an auxiliary storage configured by nonvolatile memory, for example, a hard disc drive (HDD), a solid state drive (SSD), and a flash memory, for example. The storage 140 may store a program for identifying inspection items and their reference values by accepting input of information on the food and information indicating the criteria for performing the inspection. The storage 140 may also store inspection information 141 for identifying inspection items to be inspected for each food, first reference value information 142, second reference value information 143, and third reference value information 144 indicating reference values for inspection items determined by various criteria. Details of the inspection information 141, the first reference value information 142, the second reference value information 143, and the third reference value information 144 will be described below. The data in the storage 140 may be stored in a cloud computer, and the information processing device 100 may access the cloud computer via the communication unit 110 to acquire the needed information and execute the process.

The display 150 serves to display images and texts on a monitor provided in or connected to the information processing device 100 in accordance with the instructions from the controller 130. The display 150 may, for example, display information indicating the inspection items to be inspected and their reference values for the food in accordance with the instructions from the controller 130. The input unit 120 and display 150 may be embodied as a touch panel.

The example of the configuration of the information processing device 100 is as described above.

### Data

Various types of information (data) used by the information processing device 100 to output inspection details will be described below.

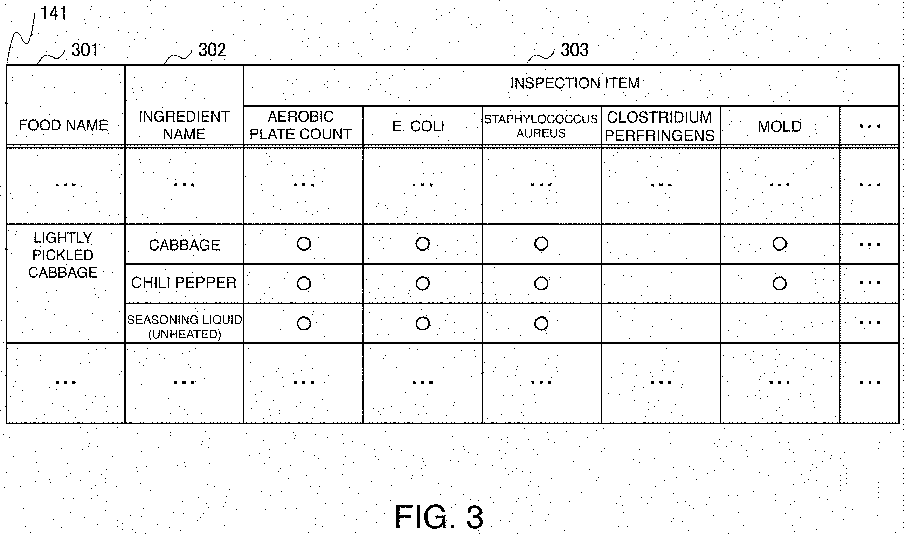

FIG. 3 illustrates a data conceptual diagram illustrating an example of a structure of the inspection information 141 stored in the storage 140. The inspection information 141 corresponds to information that specifies what must be inspected for each food.

As shown in FIG. 3, the inspection information 141 corresponds to information in which food name 301, ingredient name 302, and inspection item 303 are associated with each other.

The food name 301 is directed to information indicating the name of the food to be inspected and also identification information of the food.

The ingredient name 302 is directed to information indicating the ingredients used to create the food indicated by the corresponding food name 301, and also information indicating the components that configure the food.

The inspection item 303 is directed to information indicating whether various inspection items must be performed on each of the ingredients indicated by each ingredient name 302, that configure the corresponding food of the food name 301. FIG. 3 shows an example of an inspection item that specifies bacteria that adhere to food and can cause food poisoning or other harmful effects to customers that eat the food, but it is not limited to bacteria. In FIG. 3, marks "o" are assigned to bacteria for which inspections should be performed. The inspection items shown in FIG. 3 are only an example and may include inspection items other than those shown here.

In the example shown in FIG. 3, the ingredients for "pickled cabbage" include cabbage, chili peppers, and seasoning liquid (unheated). In the case of the "cabbage," "aerobic plate count," "E. coli," "Staphylococcus aureus," "mold," ... are listed as inspection items that should be inspected.

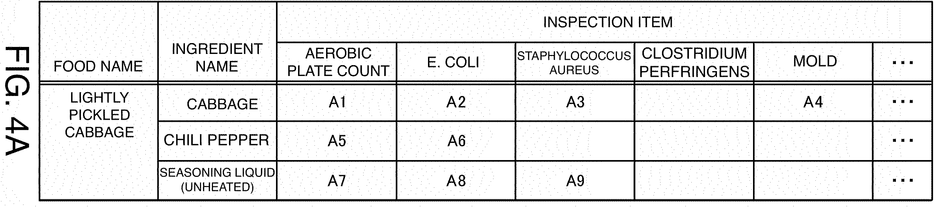

FIGS. 4A through 4C show conceptual data diagrams illustrating examples of the compositions of the first reference value information 142, second reference value information 143, and third reference value information 144, respectively. The first reference value information 142, second reference value information 143, and third reference value information 144 may each be the same in composition itself. However, the entities that established each reference value differ. The first reference value information 142 is directed to information indicating the reference value for each inspection item specified in each country. The second reference value information 143 is directed to information indicating the reference value for each inspection item specified as a global standard. The third reference value information 144 is directed to information indicating the reference value for each inspection item established by a predetermined third party, i.e., criteria other than a national or global standard. Here, the predetermined third party may be directed to, for example, a company of the store 200. Accordingly, more than one piece of first reference value information 142 may be present, and may be associated with information indicating each country to be stored in the storage 140. The first reference value information 142 need not be provided for all countries. For the third reference value information 144, the number of pieces of it may be as many as the number of predetermined third parties. If multiple predetermined third parties are present, the information processing device 100 may accept information indicating which of the pieces of the third reference value information 144 is to be used as information indicating the criteria for inspection.

The first reference value information 142, second reference value information 143, and third reference value information 144 may have the same structure as the inspection information 141, and may differ from the inspection information 141 in that the values as reference values for inspection items are stored. The first reference value information 142, second reference value information 143, and third reference value information 144 may have a different data structure than the inspection information 141, and may be directed to simply information that corresponds to the inspection items and reference values for each ingredient of the food if the reference values can be specified.

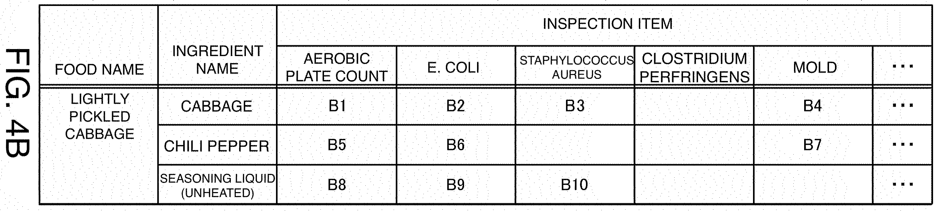

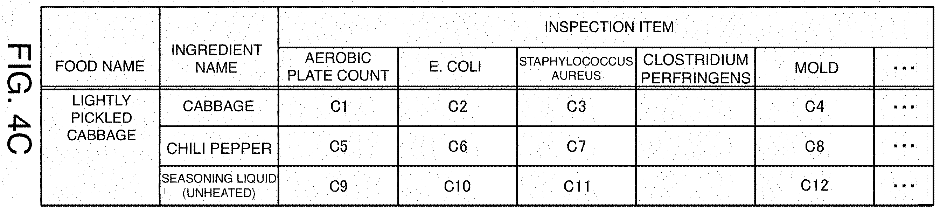

In FIG. 4A, A1 through A9 are substantially directed to information indicating numerical values as reference values, specifically, the number or amount per unit amount of bacteria that may be a problem if adhered to the food ingredients. This may also be applied to B1 to B10 in FIG. 4B and C1 to C12 in FIG. 4C.

As described above, in the storage 140 of the information processing device 100, reference values for various inspection items established by various criteria are defined, and one of these reference values is used in the actual inspection.

In addition, in the information shown in FIGS. 3, and 4A to 4C, as additional elements of information that may influence the inspection items and their corresponding reference values, information such as the state of the ingredient (e.g., heated, unheated, and frozen, for example) may further be associated with each food or ingredient and included; and the reference values may be configured to be derivable corresponding to the respective elements. For example, the time points and frequency at which the inspections should be performed, inspection methods, inspection standards, and inspection organizations may also be registered, and these matters may also be specified for the inspection items.

The inspection information 141 shown in FIG. 3 allows the information processing device 100 to identify the inspection items that must be inspected, and the respective reference value information shown in FIGS. 4A through 4C allows the information processing device 100 to identify the reference values in the inspection of each inspection item.

### Operation

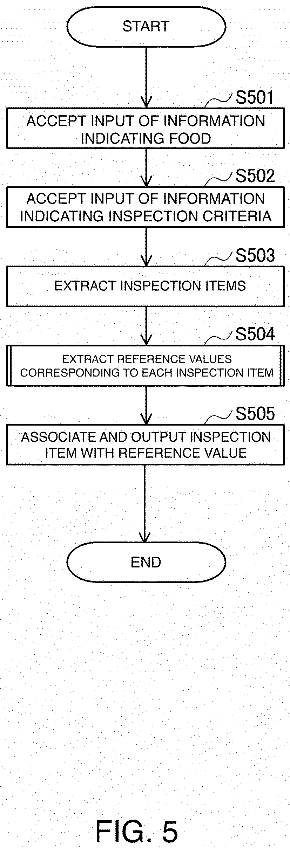

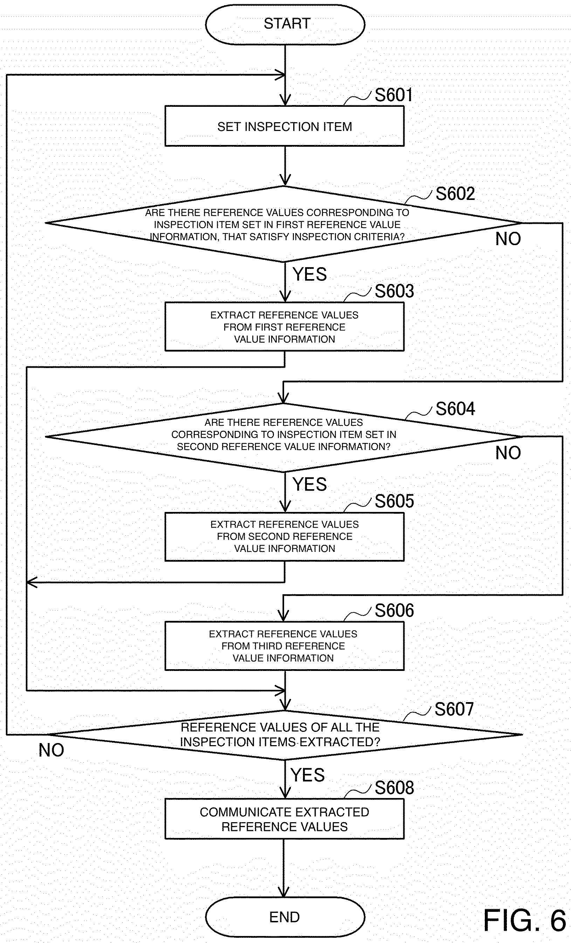

With reference to the flowcharts shown in FIGS. 5 and 6, the operation of the information processing device 100 will be described below. FIG. 5 shows a main flowchart illustrating an example of the processing for outputting information related to food inspection of the information processing device 100. FIG. 6 shows a sub-flowchart illustrating an example of processing for the extraction of reference values for the information processing device.

As shown in FIG. 5, the first receiver 131 of the controller 130 of the information processing device 100 accepts input of the information on the food to be inspected (step S501). The first receiver 131 communicates the accepted information on the food to the first extractor 133.

The second receiver 132 of the controller 130 of the information processing device 100 accepts input of the information indicating the inspection criteria (step S502). The second receiver 132 communicates the accepted information indicating the inspection criteria to the second extractor 134.

The first extractor 133 of the controller 130 of the information processing device 100 identifies from the inspection information 141 the food corresponding to the information on the food communicated by the first receiver 131, and extracts the information on the inspection items to be performed that are associated in the inspection information 141 (step S503). The first extractor 133 communicates the extracted inspection items to the second extractor 134.

The second extractor 134 of the controller 130 of the information processing device 100 extracts a reference value for each of the inspection items communicated from the first extractor 133 (step S504). The second extractor 134 extracts a reference value corresponding to the inspection item by referring to the first reference value information 142, the second reference value information 143, and the third reference value information 144, which are identified on the basis of the inspection criteria communicated from the second receiver 132. With reference to the flowchart in FIG. 6, the details of the processing of the second extractor 134 will be described below. The second extractor 134 associates and communicates the inspection items with the reference values for each extracted inspection item to the output unit 135.

The output unit 135 of the controller 130 of the information processing device 100 generates and outputs the inspection target information in which the inspection items communicated from the second extractor 134 are associated with their reference values. The output of the inspection target information by the output unit 135 is basically embodied in the form of transmission via the communication unit 110 to the information processing terminal of the store 200, but is not limited to this. This may be achieved by displaying the information on the display 150 or, although not shown in FIG. 2, by audio output from a speaker or other device provided in the information processing device 100.

As described above, the information processing device 100 can notify the stores, for example, that perform inspections of the inspection items to be performed of the inspection items to be performed and their reference values in accordance with the criteria in accordance with the location where the inspections are to be performed.

Next, with reference to FIG. 6, the details of the processing in step S504 of FIG. 5 will be described. That is, the flowchart in FIG. 6 shows the subflow of step S504 in FIG. 5.

As shown in FIG. 6, the second extractor 134 sets the inspection item to be subject to identifying a corresponding reference value from among one or more inspection items communicated from the first extractor 133 (step S601).

The second extractor 134 determines whether the reference value for the set inspection item is registered in the first reference value information 142 (step S602). If the set inspection item is registered (YES in step S602), the reference value is extracted as the reference value for the set inspection item, and the process moves to step S607. If the set inspection item is not registered (NO in step S602), the second extractor 134 determines whether the reference value for the set inspection item is registered in the second reference value information 143 (step S604).

If the reference value for the set inspection item is registered in the second reference value information 143 (YES in step S604), the second extractor 134 extracts the reference value and moves to the process in step S607. If the reference value for the set inspection item is not registered (NO in step S604), the reference value for the set inspection item is extracted from the third reference value information 144 (step S606).

When the second extractor 134 extracts the reference value for the set inspection item, it determines whether all the reference values for all the inspection items extracted by the first extractor 133 have been extracted (step S607). If all the reference values are not extracted (NO in step S607), the process returns to step S601 and the extraction process of the reference value for the next inspection item is performed. If all the reference values for the inspection items have been extracted (YES in step S607), each inspection item and the corresponding extracted reference value are communicated to the output unit 135 (step S608), and the process is terminated.

Using the first reference value information 142, second reference value information 143, and third reference value information 144 shown in FIGS. 4A through 4C as examples, the reference values identified for the lightly pickled cabbage will be described.

For example, in the case of the pickled cabbage chili peppers, according to the inspection information 141 in FIG. 3, the "aerobic plate count," "E. coli," "Staphylococcus aureus," and "mold" would be inspected, and the reference value for each would be needed.

As mentioned above, since the first reference value information 142 is given priority, the second extractor 134 extracts the reference values "A5" and "A6" for the corresponding "aerobic plate count" and "E. coli" since these reference values are registered, respectively. In contrast, in the example shown in FIG. 4A, the reference values for the "Staphylococcus aureus" and "mold" corresponding to the "chili pepper" are not registered in the first reference value information 142.

Accordingly, the reference values of the second reference value information 143, which is given priority next, is used. According to the second reference value information 143 shown in FIG. 4B, for the "chili pepper," the reference value "B7" is registered for the "mold" although no reference value is registered for the "Staphylococcus aureus." Accordingly, the second extractor 134 extracts "B7" as the reference value for the "mold" inspection.

For the "Staphylococcus aureus," the second extractor 134 extracts the reference value "C7" registered in the third reference value information 144 shown in FIG. 4C.

As a result, for example, the chili peppers used for the pickled cabbage would be inspected for the "aerobic plate count," "E. coli," "Staphylococcus aureus," and "mold," and the reference values to be used would be "A5," "A6," "C7," and "B7."

The example of the operation of the information processing device 100 is as described above.

### Summary

As shown in the above embodiments, the information processing device 100 is capable of identifying the inspection items to be performed on the food to be inspected, selecting appropriate criteria from among multiple criteria for each of the inspection items, and identifying and outputing corresponding reference values for the inspection items. Therefore, the details of the inspections can be communicated to the store 200, for example, without needing a sanitation specialist or other expert, and each store 200 can perform the appropriate inspections.

### Variations

Although one aspect of the present invention is described in the embodiments above, the ideas of the present invention are not limited to this. Various variations included as the ideas of the present invention will be described below.
(1) In the embodiments above, the information processing terminal in each store 200 inquires the information processing device 100 about the inspection items and their reference values, and the information processing device 100 responds to the inquiry, enabling each store 200 to recognize the inspection items to be inspected and the reference values. However, embodiments are not limited to this. The information processing device 100 may be provided at each store. In this case, the processing load of the information processing device 100 is reduced since the processing of the information processing device 100 is limited to each store in comparison to that in the above embodiments.
(2) In the above embodiments, the information processing terminal of each store may include a positioning system (i.e., GNSS, and GPS, for example) for positioning the location of its own terminal. When the information processing device 100 is provided at each store 200, the information processing device 100 may include a positioning system. The second receiver 132 of the controller 130 may then accept input of the current location information (latitude/longitude information) as measured by the positioning system as the information indicating the inspection criteria. This configuration eliminates the need for the user to enter the information indicating the inspection criteria at the information processing terminal of the store 200 or the information processing device 100, thereby reducing the time and efforts of the user.
(3) The information processing device 100 shown in the above embodiments may correspond to the stores in various countries as shown in FIG. 1. Accordingly, the output unit 135 of the information processing device 100 may be configured to translate and output the contents of the output in accordance with the country of each store where the inspection items and reference values are output. Well known automatic translation software may be used for the translation.
(4) In the above embodiments, the example is shown in which the information processing device 100 simply extracts the reference value corresponding to the inspection item from each reference value information (see FIGS. 4A to 4C). The information processing device 100 may output the values computed by performing computations, for example, on the reference values in accordance with predetermined conditions as the reference values for the inspections. The information processing device 100 may, for example, compute and output the reference values by performing an operation that applies a correction on the basis of the environment (e.g., temperature and humidity) at the store 200.
(5) In the above embodiments, the functional units of the information processing device 100 may be embodied by logic circuits (hardware) or dedicated circuits formed in integrated circuits (IC) chips and large scale integration (LSI), for example, or by software using central processing unit (CPU) and memory. Each functional unit may be embodied by one or more integrated circuits, and the functions of multiple functional units may be embodied by a single integrated circuit. LSIs are sometimes called VLSIs, Super LSIs, and Ultra LSIs, for example, depending on the level of integration. The term "circuit" may include the meaning of digital processing by a computer, i.e., as a functional process by software. The circuit may also be embodied by a reconfigurable circuit (e.g., field programmable gate array FPGA).
   When each functional unit of the information processing device 100 is embodied by software, the information processing device 100 includes a CPU that executes the instructions of a program to identify the inspection items and their reference values as software that embodies each function, a read only memory (ROM) or storage (referred to as "recording media") in which the input program above and various data are recorded in a readable manner by a computer (or CPU), and a random access memory (RAM) that develops the input program above, for example. The object of the present invention is achieved by a computer (or CPU) reading and executing the above programs from the above recording media. As the recording media above, "non-transient tangible media" such as tapes, disks, cards, semiconductor memory, and programmable logic circuits may be used. The input program above may be supplied to the computer above via any transmission medium (e.g., communication network, and broadcast wave) capable of transmitting the input program. The invention can also be embodied in the form of data signals embedded in a carrier wave, in which the input program above is embodied by electronic transmission.
(6) The contents shown in each configuration and variant in the above embodiments can be combined as appropriate, and the processing of the information processing device 100 can be modified as appropriate if the inspection items and corresponding reference values can be identified. For example, the processing order of step S502 and step S503 may be reversed, step S503 may be executed before step S502 is executed, or both steps may be executed in parallel. Description of Reference Numerals

100: Information Processing Device
110: Communication Unit
120: Input Unit
130: Controller
131: First Receiver
132: Second Receiver
133: First Extractor
134: Second Extractor
135: Output Unit
140: Storage
141: Inspection Information
142: First Reference Value Information
143: Second Reference Value Information
144: Third Reference Value Information
150: Display
200: Store
300: Network

## Claims

1. An information processing device comprising:
a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria;
a first receiver configured to receive an input of information indicating a food;
a second receiver configured to receive an input of information indicating a criterion for performing an inspection;
a first extractor configured to extract, on the basis of the food received by the first receiver and the inspection information, the inspection items to be inspected for the food;
a second extractor configured to extract, on the basis of the criterion received by the second receiver and the reference value information corresponding to the criterion, a respective reference value for each of the inspection items extracted by the first extractor; and
an output unit configured to output inspection target information in which the inspection items extracted by the first extractor are associated with the reference values of the inspection items extracted by the second extractor.

2. The information processing device according to claim 1, wherein
the pieces of criteria include a first criterion defined by each country, a second criterion defined as a global standard, and a third criterion defined by a predetermined third party,
the pieces of reference value information include first reference value information on the basis of the first criterion, second reference value information on the basis of the second criterion, and third reference value information on the basis of the third criterion, and
the second extractor extracts the reference values corresponding to the inspection items extracted by the first extractor on the basis of a priority order defined in an order of the first reference value information, the second reference value information, and the third reference value information.

3. The information processing device according to claim 2, wherein
the second receiver receives input of information on a location where an inspection is to be performed as the information indicating the criterion, and
the second extractor extracts a reference value of the first reference value information if the first reference value information corresponding to the information on the location where the inspection is to be performed is available, and extracts a reference value of the second reference value information or third reference value information if the first reference value information is not available.

4. The information processing device according to claim 3, further comprising:
a positioning unit configured to acquire a current location, wherein
the second receiver accepts information indicating the current location acquired by the positioning unit as information indicating a criterion for performing the inspection.

5. The information processing device according to any one of claims 1 to 4, wherein
a component of the food is an ingredient used in the food,
the inspection item is information indicating a type of bacteria to be inspected for each ingredient, and
the reference value is information indicating a number or amount of the bacteria per unit.

6. An information processing method causing
a computer having access to a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria to perform:
a first reception step for receiving an input of information indicating a food;
a second reception step for receiving an input of information indicating a criterion for performing an inspection;
a first extraction step for extracting, on the basis of the food received in the first reception step and the inspection information, the inspection items to be inspected for the food;
a second extraction step for extracting, on the basis of the criterion received in the second reception step and the reference value information corresponding to the criterion, a respective reference value for each of the inspection items extracted in the first extraction step; and
an outputting step for associating and outputting the inspection items extracted in the first extraction step with the reference values of the inspection items extracted in the second extraction step.

7. An information processing program causing
a computer having access to a storage configured to store inspection information in which a food is associated with inspection items to be inspected for components configuring the food; and a plurality of pieces of reference value information, each of which defines a reference value to be satisfied for the corresponding inspection item defined in accordance with each of a plurality of mutually different criteria to embody:
a first reception function of receiving an input of information indicating a food;
a second reception function of receiving an input of information indicating a criterion for performing an inspection;
a first extraction function of extracting, on the basis of the food received by the first reception function and the inspection information, the inspection items to be inspected for the food;
a second extraction function of extracting, on the basis of the criterion received by the second reception function and the reference value information corresponding to the criterion, a respective reference value for each of the inspection items extracted by the first extraction function; and
an outputting function of associating and outputting the inspection items extracted by the first extraction function with the reference values of the inspection items extracted by the second extraction function.
